# EUROPÄISCHE PATENTANMELDUNG

(11) **EP 0 928 602 A2**
(43) Veröffentlichungstag der Anmeldung: **14.07.1999**
(21) Anmeldenummer: 99106720.8
(22) Anmeldetag: 20.01.1997
(51) Int. Cl.: A61B 17/58

(54) **Scherwerkzeug für Implantate**

(30) Priorität: 24.01.1996 CH 18696
(62) Teilanmeldung aus: 97100775.2
(71) Anmelder: Hermann, Werner, 6312 Steinhausen (CH)
(72) Erfinder: Hermann, Werner, 6312 Steinhausen (CH); Matzen, Klaus A., Prof. Dr. Dr., 86199 Augsburg-Göggingen (DE)
(74) Vertreter: Hotz, Klaus, Dipl.-El.-Ing./ETH Patentanwalt

(57) **Zusammenfassung**

**Scherwerkzeug**. Das Scherwerkzeug (**100**) umfasst einen zylindermantelförmigen Aussenscherkörper (**120**) und einen darin drehbar angeordneten Innnenscherkörper (**140**). Der Aussenscherkörper (**120**) weist einen Boden (**124**) mit Bodendurchbruch (**126**) auf, und der Innenscherkörper (**140**) weist einen Längsdurchbruch (**146**) auf. Aussenscherkörper (**120**) und Innenscherkörper (**140**) sind vor dem Schervorgang so angeordnet, dass der Bodendurchbruch (**126**) und der Längsdurchbruch (**146**), in welchen das zu scherende Teil aufgenommen ist, fluchten. Während des Schervorganges werden der Aussenscherkörper (**120**) und der Innenscherkörper (**140**) relativ zueinander gedreht, bis der Bodendurchbruch (**126**) und der Längsdurchbruch (**146**) nicht mehr fluchten. Aussenscherkörper (**120**) und Innenscherkörper (**140**) sind mittels einer Fixiereinrichtung (**150, 160, 170**) gegen relative Bewegungen in Richtung der Längsachse (**A**) gesichert; vorzugsweise ist die Fixiereinrichtung (**150, 160, 170**) werkzeugfrei lösbar.

## Beschreibung

Die Erfindung betrifft ein Scherwerkzeug nach dem Oberbegriff des Anspruchs **1**.

Längliche Teile bzw. Bolzenenden, die mit Hilfe solcher Scherwerkzeuge abgeschert werden, finden sich an Implantaten, beispielsweise an Pedikelschrauben, welche bei krankhafter Veränderung wie Spondylose an Wirbelkörpern und Bandscheiben verwendet werden, um die Stellung der Wirbelkörper zu korrigieren und die durch die Korrektur hergestellte relative Lage der betroffenen Wirbelkörper zu fixieren. Ein beim Einsetzen von Implantaten auftretendes Problem besteht darin, dass gewisse Bestandteile, insbesondere Bolzen, beispielsweise von Pedikelschrauben, nicht vor dem operativen Eingriff in der richtigen Abmessung gefertigt werden können sondern nach ihrer Implantierung abgelängt werden müssen. Die Stammanmeldung der vorliegenden Teilanmeldung, ***EP-97 100 755*** beschreibt beispielsweise ein Implantat, bei dessen Implantierung ein Ablängen vorzunehmen ist.

Der Ablängvorgang muss sich so abspielen, dass sich weder beim Ablängen selbst noch anschliessend daran unerwünschte Belastungen im Knochen und im Bolzen ergeben; der Bolzen soll insbesondere dabei nicht oder nur geringfügig tordiert werden. Es hat sich als vorteilhafter erwiesen, die Bolzen nicht durch Absägen sondern durch Abscheren abzulängen. Das zum Abscheren verwendete Scherwerkzeug muss möglichst einfach betätigbar sein und keinen grossen Kraftaufwand erfordern. Erwünscht ist dabei, dass die Deformationsarbeit am Bolzen möglichst klein ist und dass die scherenden Kanten oder Flächen über möglichst lange Hebelarme betätigbar sind, damit die zur Scherung aufzuwendende Kraft nicht zu gross ist Gleichzeitig soll der Bereich des Scherwerkzeuges, der proximal liegt, möglichst klein sein.

Der Erfindung liegt die Aufgabe zugrunde, ein für die Verwendung an Implantaten optimales Scherwerkzeug der eingangs genannten Art zu schaffen, welches einwandfreie Scherungen erlaubt und leicht betätigbar ist.

Diese Aufgabe wird erfindungsgemäss durch die Merkmale des kennzeichnenden Teils des Anspruchs **1** gelöst. Vorteilhafte Weiterbildungen des erfindungsgemässen Scherwerkzeuges sind durch die vom Anspruch **1** abhängigen Ansprüche **2** bis **10** definiert.

Das erfindungsgemäss Scherwerkzeug umfasst einen zylindermantelförmig ausgebildeten Aussenscherkörper mit einem Boden und einen im letzteren koaxial angeordneten, zylindrisch ausgebildeten Innenscherkörper. Der Innenscherkörper ist in Richtung der Längsachse kürzer als der Aussenscherkörper und so angeordnet, dass er nicht ganz bis zum Boden des Aussenscherkörpers reicht. Der Innenscherkörper und der Aussenscherkörper bilden einen Gleitsitz, so dass sie relativ zueinander gedreht werden können. In den Innenscherkörper ist ein sich über seine Länge erstreckender Längsdurchbruch eingearbeitet, der parallel zur Längsachse verläuft, jedoch exzentrisch angeordnet ist und einen gewissen Durchbruchs-Abstand von der Längsachse aufweist. Im gleichen Durchbruchs-Abstand von der Längsachse ist in den Boden des Aussenscherkörpers ein Bodendurchbruch eingearbeitet. Der eigentliche Scherbereich bzw. das Scherspiel befindet sich zwischen der Endfläche des Innenscherkörpers und der inneren Bodenfläche des Aussenscherkörpers.

Die Wirkungsweise dieses Scherwerkzeuges ist wie folgt: Vor dem Abscheren des Bolzens werden der Innenscherkörper und der Aussenscherkörper in eine Anfangslage gebracht, in welcher der Längsdurchbruch des Innenscherkörpers mit dem Bodendurchbruch des Aussenscherkörpers fluchtet. Sodann wird der Bolzen mit seinem abzulängenden Ende soweit in den in den Längsdurchbruch des Innenscherkörpers eingeführt bzw. das Scherwerkzeug mit dem Innenscherkörper soweit über das abzulängende Ende des Bolzens gestülpt, dass die Endpartie des Bolzens durch den Bodendurchbruch des Aussenscherkörpers in den Längsdurchbruch des Innenscherkörpers ragt und der Bolzen mit demjenigen Querschnitt, der nach dem Abschervorgang seinen Endquerschnitt bildet, in der Höhe der Innenfläche Bodens des Aussenscherkörpers bzw. in der Höhe des eigentlichen Scherbereiches liegt. Anschliessend werden der Aussenscherkörper und der Innenscherkörper relativ zueinander in eine Endlage gedreht, wobei der am Implantat verbleibende Bereich des Bolzens durch den Aussenscherkörper und der abzuscherende Bereich des Bolzens durch den Innenscherkörper geführt ist. Während der Relativdrehung von Innenscherkörper und Aussenscherkörper wird der Bolzen in der Höhe des eigentlichen Scherbereiches zertrennt.

Da es gelegentlich vorkommt, dass während des Schervorganges eine Verklemmung, beispielsweise des Bolzens im Scherwerkzeug, eintritt, ist es vorteilhaft, zur gegenseitigen Fixierung von Innenscherkörper und Aussenscherkörper nicht eine Fixierschraube zu verwendet, zu deren Lösung ein Schraubenschlüssel benötigt würde; ein solches Ausschrauben wäre umständlich, und es bestünde - da der Schraubenschlüssel und das Scherwerkzeug dabei gehalten werden müssten - die Gefahr, dass die gelöste Fixierschraube nicht rechtzeitig gepackt werden kann und in die Operationswunde fällt. Bei einer vorteilhaften Weiterbildung des neuen Scherwerkzeuges erfolgt die gegenseitige Fixierung von Innenscherkörper und Aussenscherkörper mit Hilfe einer Fixiereinrichtung mit einem Schieberteil, das zwischen einer Fixierstellung und einer Lösestellung hin- und her schiebbar ist. Befindet sich das Schieberteil in seiner Fixierstellung, so sind Innenscherkörper und Aussenscherkörper so aneinander fixiert, dass sie relativ zueinander drehbar, aber in axialer Richtung nicht gegeneinander verschiebbar sind. Befindet sich das Schieberteil in seiner Lösestellung, so kann der Innenscherkörper relativ zum Aussenscherkörper nicht nur gedreht sondern auch in axialer Richtung bewegt und aus dem Aussenscherkörper gezogen werden. Zur Betätigung des Schieberteils ist ein Drückerelement vorgesehen. Zur Aufnahme des Schieberteils sind auf einer bestimmten axialen Höhe, vorzugsweise in der Nähe der Ansatzquerschnitte der Betätigungshebel, im Aussenscherkörper eine schlitzartige Öffnung und an der Oberfläche des Innenscherkörpers eine sich über einen Teil des Umfanges erstreckende nutartige Ausnehmung vorgesehen. In der Fixierstellung greift das im Aussenscherkörper gehaltenen Schieberteil in die Ausnehmung des Innenscherkörpers; das Drückerteil befindet sich hierbei in einer äusseren Position. In der Lösestellung gibt das Schieberteil den Innenscherkörper für eine Bewegung in Richtung der Längsachse relativ zum Aussenscherkörper frei; das Drückerteil befindet sich hierbei in einer inneren Position, in welche es gegen die Kraft einer Feder gedrückt wurde; hierbei lässt sich der Innenscherkörper vom Aussenscherkörper entfernen, was auch die Reinigung sowie die Kontrolle der Kanten des eigentlichen Scherbereiches vorteilhaft ist.

Weitere Einzelheiten und Vorteile des erfindungsgemässen Scherwerkzeuges werden im folgenden anhand eines Ausführungsbeispiels und mit Bezug auf die Zeichnung ausführlich beschrieben. Es zeigen:
- **Fig. 1**: ein Ausführungsbeispiel des erfindungsgemässen Scherwerkzeuges, in Seitenansicht;
- **Fig. 2A**: einen der Betätigungshebel des in **Fig. 1** dargestellten Scherwerkzeuges, in Draufsicht;
- **Fig. 2B**: den in **Fig. 2A** dargestellten Betätigungshebel, in Seitenansicht;
- **Fig. 2C**: der gemäss **Fig. 1** untere Betätigungshebel mit einem ersten Element einer Anschlagvorrichtung, von oben;
- **Fig. 2D**: der gemäss **Fig. 1** obere Betätigungshebel mit einem zweiten Element der Anschlagvorrichtung, von unten;
- **Fig. 2E**: die beiden Betätigungshebel mit zusammenwirkenden Elementen der Anschlagvorrichtung, im Schnitt;
- **Fig. 3A**: den Aussenscherkörper des in **Fig. 1** dargestellten Scherwerkzeuges, in Seitenansicht;
- **Fig. 3B**: den in **Fig. 3A** dargestellten Aussenscherkörper in einem einem Vertikalschnitt;
- **Fig. 3C**: den in den **Fig. 3A, 3B** dargestellten Aussenscherkörper, in Draufsicht, teilweise geschnitten;
- **Fig. 4A**: den Innenscherkörper des in **Fig. 1** dargestellten Scherwerkzeuges, in Seitenansicht;
- **Fig. 4B**: den in **Fig. 4A** dargestellten Innenscherkörper, in einem Vertikalschnitt;
- **Fig. 4C**: den in den **Fig. 4A, 4B** dargestellten Innenscherkörper in Draufsicht, teilweise geschnitten;
- **Fig. 5A**: den Deckel des in **Fig. 1** dargestellten Scherwerkzeuges, in Seitenansicht, von innen;
- **Fig. 5B**: den in **Fig. 5A** dargestellten Deckel, in einem Horizontalschnitt;
- **Fig. 5C**: den in den **Fig. 5A, 5B** dargestellten Deckel, in einem weiteren Schnitt;
- **Fig. 6A**: das Schieberteil des in **Fig. 1** dargestellten Scherwerkzeuges, von der Seite;
- **Fig. 6B**: das in **Fig. 6A** dargestellte Schieberteil, in einem Horizontalschnitt; und
- **Fig. 7**: das Drückerteil des in **Fig. 1** dargestellten Scherwerkzeuges.

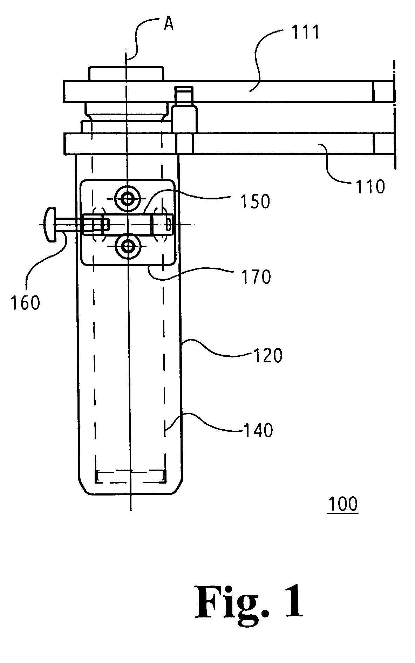

Gemäss **Fig.1** umfasst das erfindungsgemässe Scherwerkzeug **100** zum Ablängen von länglichen Teilen, beispielsweise von Bolzen von Pedikelschrauben, zwei Betätigungshebel **110, 111**, einen Aussenscherkörper **120**, einen Innenscherkörper **140**, ein Schieberteil **150**, ein Drückerteil **160** und einen Deckel **170**.

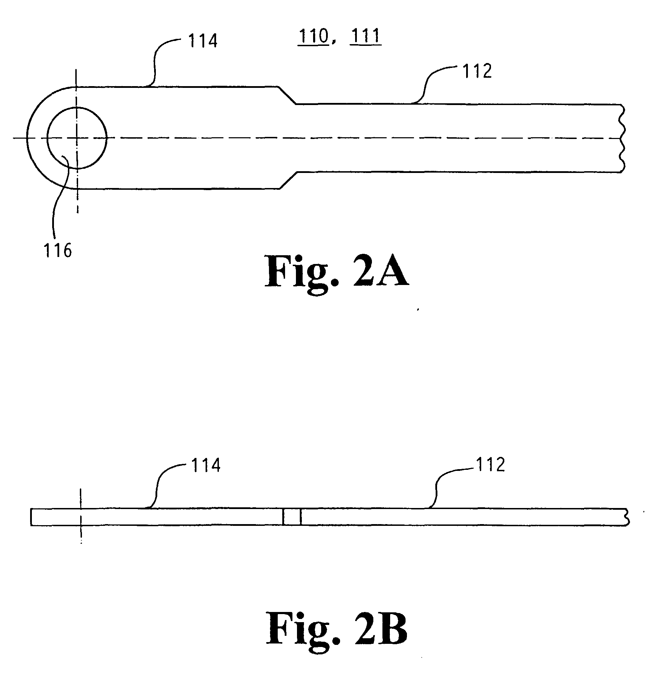

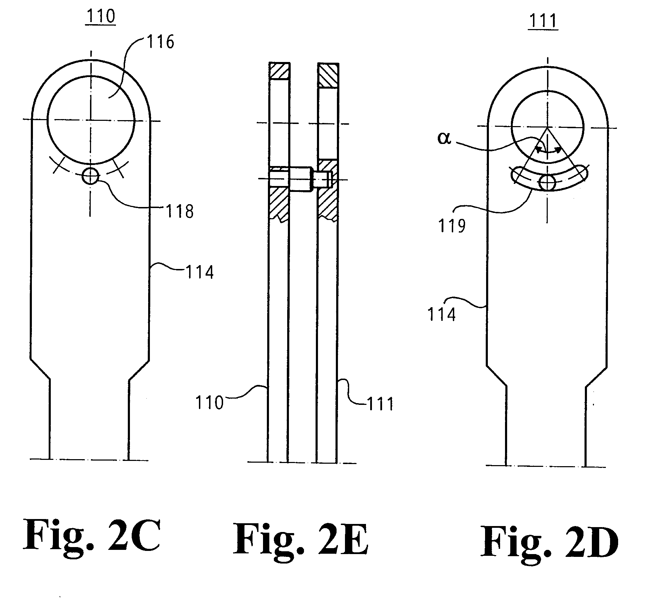

Jeder der Betätigungshebel **110**, **111** weist gemäss **Fig. 2A, 2B** einen nicht in ganzer Länge dargestellten Griffteil **112** und einen Befestigungsteil **114** mit einer Bohrung **116** auf, welche zur Aufnahme des Aussenscherkörpers **120** bzw. des Innenscherkörpers **140** bestimmt ist. Die Griffteile **112** der Betätigungshebel **110, 111** sind verhältnismässig lang, damit die zur Abscherung der bolzenartigen Teile aufzuwendende Kraft nicht allzu gross sein muss. Gemäss **Fig. 2C** weisen der untere Betätigungshebel **110** einen Nocken **118** und gemäss **Fig**. **2D** der obere Betätigungshebel **111** eine kreisbogenförmige, sich über einen Öffnungswinkel α erstreckende Nut **119** auf, in welche im montierten Zustand des Scherwerkzeuges **100** gemäss **Fig. 2E** zusammenwirken, wobei der Nocken **118** in die Nut **119** eingreift. Die Anordnung des Nockens **118** und der Nut **119** sind so, dass die Betätigungshebel **110, 111** aus einer Anfangslage, in welcher sie um den Winkel α voneinander weggedreht sind, in eine Endlage, in welcher sie übereinander bzw. auf demselben Radius angeordnet sind, schwenkbar sind.

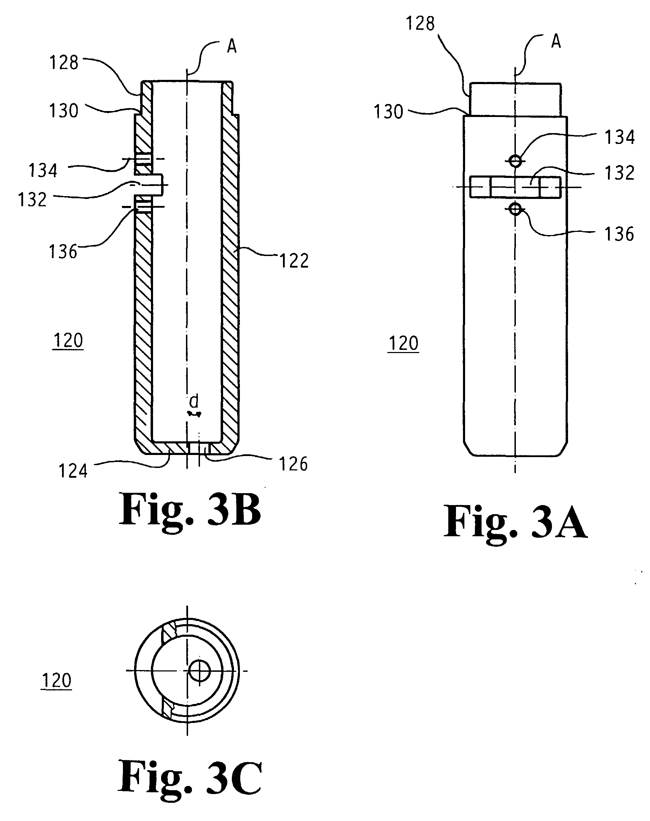

Der in den **Fig. 3A, 3B, 3C** genauer dargestellte Aussenscherkörper **120** hat im wesentlichen die Form eines Hohlzylinders mit einer Längsachse **A**, mit einer Wandung **122** und mit einem Boden **124**. Der Boden **124** weist einen exzentrisch angeordneten, parallel zur Längsachse **A** gerichteten Bodendurchbruch **126** in einem Durchbruchs-Abstand **d** von der Längsachse **A**, im allgemeinen in Form einer Bohrung, auf. Der Aussendurchmesser des Aussenscherkörpers **120** ist in dessen oberstem Bereich **128** oberhalb einer Schulter **130** verkleinert. Die Wandung **122** des Aussenscherkörpers **120** besitzt eine seitliche schlitzartige Öffnung **132** sowie zwei Bohrungen **134, 136**, die oberhalb bzw. unterhalb der schlitzartigen Öffnung **132** angeordnet sind.

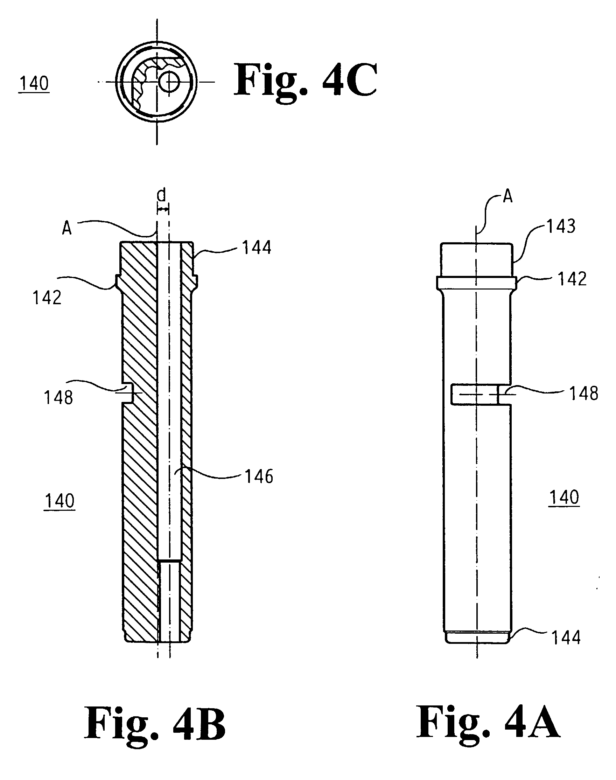

Im Aussenscherkörper **120** ist der in den **Fig. 4A. 4B, 4C** dargestellte drehkolbenartige Innenscherkörper **140** angeordnet. Der Innenscherkörper **140** weist oberhalb eines Kragens **142** einen aus dem Aussenscherkörper **120** herausragenden Kopf **143** auf, dessen Durchmesser dem Durchmesser der Bohrung **116** des zugehörigen Handgriffes **110** entspricht. Der dem Kopf **143** gegenüberliegende Endbereich **144** des Innenscherkörpers **140** weist einen verringerten Durchmesser und eine gerundete oder abgeschrägte Kante auf. Ferner weist der Innenscherkörper **140** einen in seiner Längsrichtung verlaufenden exzentrischen Längsdurchbruch **146**, ebenfalls im Durchbruchs-Abstand **d** von der Längsachse **A** auf, im allgemeinen in Form einer Längsbohrung, auf; dessen Querabmessungen und Lage sind so gewählt, dass bei geeigneter Relativlage von Innenscherkörper **140** und Aussenscherkörper **120** der Längsdurchbruch **146** des Innenscherkörpers **140** mit dem Bodendurchbruch **126** des Aussenscherkörpers **120** fluchtet. Die Anordnung ist so, dass der Längsdurchbruch **146** des Innenscherkörpers **140** und der Bodendurchbruch **126** des Aussenscherkörpers **120** dann fluchten, wenn sich die Betätigungshebel **110, 111** in ihren Anfangslagen, also um den Winkel α voneinander entfernt, befinden. Wenn die Betätigungshebel **110, 111** ihre Endlagen einnehmen, fluchten der Längsdurchbruch **146** des Innenscherkörpers **140** und der Bodendurchbruch **126** des Aussenscherkörpers **120** nicht mehr. Der Innenscherkörper **140** weist eine nutähnliche seitliche Ausnehmung **148** auf, die sich über einen Teil seines Umfanges erstreckt und die im montierten Zustand der Scherwerkzeuges **100** in gleicher axialer Höhe angeordnet ist wie die schlitzartige Öffnung **132** des Aussenscherkörpers **120**.

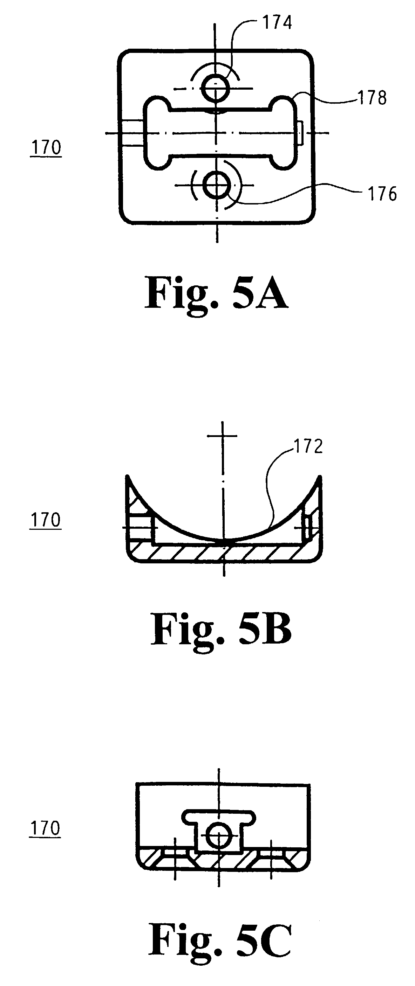

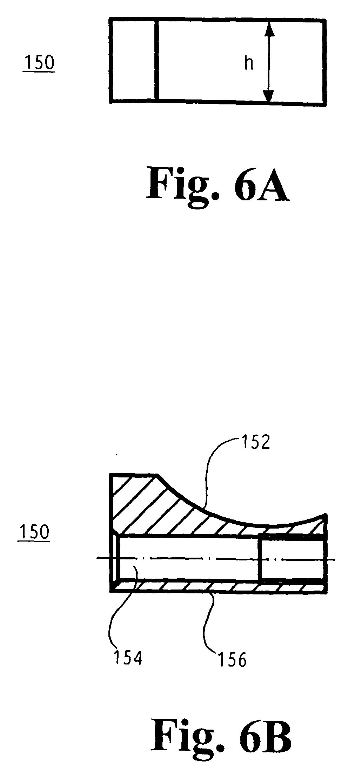

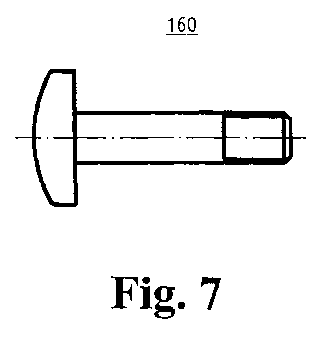

Der Aussenscherkörper **120** und der Innenscherkörper **140** sind mit Hilfe einer Fixiereinrichtung so aneinander fixiert, dass sie sich relativ zueinander drehen können, dass aber ihre relative Bewegung in Richtung der Längsachse **A** bei geeigneter Stellung der Fixiereinrichtung verunmöglicht wird. Die Fixiereinrichtung umfasst das in den **Fig. 6A, 6B** dargestelltes Schieberteil **150,** das in **Fig. 7** dargestellte Drückerteil **160** und den in den **Fig. 5A, 5B, 5C** dargestellten Deckel **170**. Die Fixiereinrichtung verbindet im scherbereiten Zustand den Aussenscherkörper **120** und den Innenscherkörper **140** derart, dass sie relativ zueinander um ihre gemeinsame Längsachse **A** rotieren können, während sie gegen lineare Relativbewegungen in Richtung der Längsachse **A** gesichert sind.

Das Schieberteil **150** weist eine zylindrisch gerundete konkave Fläche **152** auf, deren Radius dem Radius der Ausnehmung **148** des Innenscherkörpers **140** entspricht. Im weiteren weist das Schieberteil **150** eine Bohrung **154** mit Gewinde auf, welche zur Aufnahme des Drückerteils **160** bestimmt ist. Die quer zur gerundeten Fläche **152** verlaufende Höhe **h** des Schieberteils **150** entspricht der axialen Abmessung der Ausnehmung **148** des Innenscherkörpers **140** bzw. diese Höhe **h** ist um so viel geringer als die axiale Abmessung der Ausnehmung **148**, dass sich das Schieberteil **150** in der Art eines Gleitsitzes in der Ausnehmung **148** verschieben lässt.

Das Drückerteil **160** wird gemäss **Fig. 7** durch eine Spezialschraube gebildet, welche in die Bohrung **154** des Schieberteils **150** eingeschraubt ist.

Der Deckel **170** weist eine zylindermantelförmige Innenfläche **172** auf, die der Aussenfläche des Aussenscherkörpers **120** entspricht, so dass er mittels nicht dargestellten Befestigungsschrauben, für welche Bohrungen **174, 176** vorgesehen sind, satt am Aussenscherkörper **120** anliegend befestigt werden kann. In der axialen Höhe der Ausnehmung **148** des Innenscherkörpers **140** ist in der zylindermantelförmigen Innenfläche **172** des Deckels **170** eine Nut **178** eingearbeitet, welche zur Aufnahme des Schieberteils **150** bestimmt ist; das Schieberteil **150** ist dabei so angeordnet, dass die seiner gerundeten Fläche **152** gegenüberliegende, ebene Fläche **156** an den Grund der Nut **178** des Deckels **170** zu liegen kommt; die Bewegung des Schieberteils **150** relativ zum Deckel **170** ist eine hin- und hergehende lineare Bewegung, während welcher das Schieberteil **150** in der Nut **178** des Deckels **170** geführt ist. Das Schieberteil **150** und das Drückerteil **160** werden durch den mit den nicht dargestellten Deckelschrauben am Aussenscherkörper **120** befestigten Deckel **170** gehalten.

Mittels des Drückerteils **160** lässt sich das Schieberteil **150** werkzeugfrei aus seiner Fixierstellung und in seine Lösestellung und zurück schieben. In der Fixierstellung ist das Schieberteil **150** so angeordnet, dass es in der Nut **178** des Deckels **170** aufgenommen ist, durch die Öffnung **132** des Aussenscherkörpers **120** in die Ausnehmung **148** des Innenscherkörpers **140** greift und dadurch verhindert, dass sich der Innenscherkörper **140** in axialer Richtung relativ zum Aussenscherkörper **120** bewegen kann; hierbei befindet sich das Drückerteil **160** unter der Kraft einer nicht dargestellten Feder in seiner äusseren Position. In seiner Lösestellung ist das Schieberteil **150** zwar noch stets in der Nut **178** des Deckels **170** angeordnet, greift aber nicht mehr in die Ausnehmung **148** des Innenscherkörper **140** und gibt dadurch diesen für eine axiale Bewegung relativ zum Aussenscherkörper **120** frei; hierfür wird das Drückerteil **160** gegen die Kraft der Feder in seine innere Position geschoben.

Der Bodendurchbruch **126** des Aussenscherkörpers **120** und der Längsdurchbruch **146** des Innenscherkörpers **140** sind für die Aufnahme eines Teils des abzulängenden länglichen Bolzens, beispielsweise des Schaftes einer Pedikelschraube, bestimmt, und weisen vorzugsweise gleiche Querschnittsformen, im allgemeinen Kreise, auf. Im montierten Zustand ist das axiale Spiel zwischen dem Innenscherkörper **140** und dem Aussenscherkörper **120**, also das Schnittspiel, gering und kann beispielsweise etwa 0.02 bis 0.04 mm betragen. Nach der definitiven Implantierung des abzuscherenden Bolzens wird das Scherwerkzeug **100** mit fluchtenden Durchbrüchen **126, 146** so weit wie erforderlich auf den Bolzen aufgeschoben, wobei der Boden **124** des Aussenscherkörpers **120** proximal bzw. bei einem Patienten in Bauchlage unten, die Betätigungshebel **110, 111** distal bzw. bei einem Patienten in Bauchlage oben zu liegen kommen. Hierbei sind die Betätigungshebel **110, 111** um den Winkel α voneinander weggeschwenkt. Daraufhin werden die Betätigungshebel **110, 111** aufeinander zu geschwenkt, und dabei wird der abzuscherende Bolzen durch die relative gegenseitige Rotation des Aussenscherkörpers **120** einerseits und des Innenscherkörpers **140** anderseits abgeschert. Während des Schervorganges ist der mit dem Impantat verbunden bleibende Teil des Bolzens im Aussenscherkörper **120** und der abzuscherende Teil des Bolzens im Innenscherkörper **140** geführt. Die Abscherstelle befindet sich dort, wo der Bolzen aus dem Längsdurchbruch **146** des Innenscherkörpers **140** in den Bodendurchbruch **126** des Aussenscherkörpers **120** übergeht. Während des gesamten Schervorganges befindet sich das Schieberteil **150** in der Fixierstellung. Sollen der Innenscherkörper **140** und der Aussenscherkörper **120** ganz oder teilweise voneinander entfernt werden, sei es um während des Gebrauchs des Scherwerkzeuges **100** eine Verklemmung zu lösen oder um nach Gebrauch des Scherwerkzeuges **100** dieses gründlich zu reinigen, so wird das Schieberteil **150** durch Druck auf das Drückerteil **160** gegen die Kraft der Feder in seine Lösestellung gebracht und der Innenscherkörper **140** vom Aussenscherkörper **120** entfernt.

Im allgemeinen versucht man, Bolzen so weitgehend wie möglich abzuscheren. Ohnehin verbleibt jeweils ein nicht abscherbarer Teil des Bolzens, dessen Länge im wesentlichen der Dicke des Bodens des Aussenscherkörpers entspricht.

### Bezugszeichenliste Stammgesuch

- **1**: Pedikelschrauben
- **2**: Verbindungsstange
- **3**: Verbindungsstücke
- **4**: Schaft
- **5**: Gewindeteil
- **6**: Klemmkörper
- **7**: Erste Bohrung
- **8**: Spannbacke
- **9**: Spannbacke
- **10**: Schlitz
- **11**: Zweite Bohrung
- **12**: Klemmschraube
- **13**: Kopf
- **14**: Längsachse
- **15**: Bohrung
- **16**: Oberfläche
- **17**: Durchmesser
- **18**: Gebogener Teil
- **19**: Konischer Übergang
- **20**: Schaft
- **21**: Konische Vertiefung
- **22**: Spannscheibe
- **23**: Konische Vertiefung
- **24**: Rand
- **25**: Gewinde
- **26**: Mutter
- **27**: Klemmschraube
- **28**: Abflachungen
- **29**: Spannscheibe mit Langloch
- **30**: Wirbelkörper
- **35**: Scherwerkzeug
- **36**: Zylinder
- **37**: Drehkolben
- **38**: Kopf
- **39**: Bohrung
- **40**: Boden
- **41**: Weitere Bohrung

### Bezugszeichenliste Teilgesuch

- **A**: Längsachse
- **d**: Durchbruchsabstand
- **a**: Schwenkwinkel
- **h**: Höhe
- **100**: **Scherwerkzeug**
- **110**: **Betätigungshebel (Anfangslage, Endlage)**
- 111: Betätigungshebel
- 112: Griffteil
- 114: Angriffsteil
- 116: Bohrung
- 118: Nocken
- 119: Nut
- **120**: **Aussenscherkörper**
- 122: Wandung
- 124: Boden
- 126: Bodendurchbruch
- 128: Bereich
- 130: Schulter
- 132: schlitzartige Öffnung
- 134: Bohrung
- 136: Bohrung
- **140**: **Innenscherkörper**
- 142: Kragen
- 143: Kopf
- 144: Endteil
- 145: Kante
- 146: Längsdurchbruch
- 148: nutartige Ausnehmung
- **150**: **Schieberteil (Fixierstellung, Lösestellung)**
- 152: gerundete Begrenzungsfläche
- 154: Bohrung
- 146: Fläche
- **160**: **Drückerteil (innere Position, äussere Position)**
- **170**: **Deckel**
- **172**: zyl. Innenfläche
- **174**: Bohrung
- **176**: Bohrung
- **178**: Nut

## Patentansprüche

1. Scherwerkzeug (**100**) zum Abscheren eines länglichen Teils eines Implantates,
***dadurch gekennzeichnet, dass***
es
- einen zylindermantelförmigen Aussenscherkörper (**120**) mit einem Boden (**124**), welcher einen exzentrischen Bodendurchbruch (**126**) in einem Durchbruchs-Abstand (**d**) von der Längsachse (**A**) des Aussenscherkörpers(**120**) und
- einen zylindrischen Innenscherkörper (**120**) umfasst, der im Aussenscherkörper (**140**) angeordnet ist und einen exzentrischen Längsdurchbruch (**146**) im gleichen Durchbruchs-Abstand (**d**) von der Längsachse (**A**) besitzt, wobei
- der Aussenscherkörper (**120**) und der Innenscherkörper (**140**) relativ zueinander aus einer Anfangslage, in welcher der Bodendurchbruch (**126**) des Aussenscherkörpers (**120**) und der Längsdurchbruch (**146**) des Innenscherkörpers (**140**), die zur Aufnahme des zu zerscherenden Teils bestimmt sind, fluchten, in eine Endlage drehbar sind, in welcher der genannte Bodendurchbruch (**126**) und der genannte Längsdurchbruch (**146**) nicht fluchten, und wobei
- eine Fixiervorrichtung (**150, 160, 170**) vorgesehen ist, die den Aussenscherkörper (**120**) und den Innenscherkörper (**140**) gegen relative Bewegungen in Richtung der Längsachse (**A**) sichert.

2. Scherwerkzeug (**100**) nach Anspruch **1**,
***dadurch gekennzeichnet, dass***
die Fixiereinrichtung (**150, 160, 170**) vorzugsweise werkzeugfrei lösbar ist, indem sie aus ihrer Fixierstellung, in welcher sie den Innenscherkörper (**140**) und den Aussenscherkörper (**120**) gegen relative Bewegungen in Richtung der Längsachse (**A**) sichert, werkzeugfrei in eine Lösestellung bringbar ist, in welcher sie den Innenscherkörper (**140**) für eine relative Bewegung zum Aussenscherkörper (**120**) in Richtung der Längsachse (**A**) freigibt.

3. Scherwerkzeug (**100**) nach Anspruch **2,**
***dadurch gekennzeichnet, dass***
die Fixiereinrichtung ein schiebbar am Aussenscherkörper (**120**) angeordnetes Schieberteil (**150**) umfasst, das in der Fixierstellung in eine Ausnehmung (**148**) des Innenscherkörpers (**140**) eingreift und in der Lösestellung ausserhalb der Ausnehmung (**148**) des Innenscherkörpers (**140**) angeordnet ist.

4. Scherwerkzeug (**100**) nach Anspruch **3,**
**dadurch gekennzeichnet, dass**
die Fixiereinrichtung ein am Schieberteil (**150**) befestigtes Drückerteil (**160**) umfasst, das quer zur Längsachse (**A**) verschiebbar ist um das Schieberteil (**150**) dabei zwischen der Fixierstellung und der Lösestellung zu verschieben ist, wobei sich das Drückerteil (**160**) vorzugsweise in der Wirkstellung in einer äusseren und in der Lösestellung in einer inneren Position befindet.

5. Scherwerkzeug (**100**) nach Anspruch **4,**
***dadurch gekennzeichnet, dass***
sich das Drückerteil (**160**) gegen die Kraft einer Feder aus der Fixierstellung in die Lösestellung schieben lässt.

6. Scherwerkzeug (**100**) nach Anspruch **4,**
***dadurch gekennzeichnet, dass***
das Schieberteil (**150**) und das Drückerteil (**160**) durch einen Deckel (**170**) gehalten sind, welcher am Aussenscherkörper (**140**) anliegend befestigt ist und dessen Innenfläche eine Nut (**178**) zur Führung des Schieberteils (**150**) besitzt.

7. Scherwerkzeug (**100**) nach Anspruch **1,**
***dadurch gekennzeichnet, dass***
am Aussenscherkörper (**120**) und am Innenscherkörper (**140**), vorzugsweise an den Betätigungshebeln (**110, 111**) eine Anschlagvorrichtung (**118, 119**) angeordnet ist, welche die Anfangslage und die Endlage definiert.

8. Scherwerkzeug (**100**) nach Anspruch **7,**
***dadurch gekennzeichnet, dass***
die Anschlagvorrichtung einen Nocken (**118**) an einem der Betätigungshebel (**110**) und eine kreisbogenförmige, konzentrisch zur Längsachse (**A**) angeordnete, sich über einen Winkel (a) erstreckende Nut (**119**) am anderen der Betätigungshebel (**111**) umfasst, in welcher der Nocken (**118**) verschiebbar aufgenommen ist.

9. Scherwerkzeug (**100**) nach Anspruch **1,**
***dadurch gekennzeichnet, dass***
der Längsdurchbruch (**146**) des Innenscherkörpers (**140**) und der Bodendurchbruch (**126**) des Aussenscherkörpers (**120**) denselben Querschnitt aufweisen.

10. Scherwerkzeug (**100**) nach Anspruch **1,**
***dadurch gekennzeichnet, dass***
zwischen der Endfläche des Innenscherkörpers (**140**) und der Innenfläche des Bodens (**124**) des Aussenscherkörpers (**120**) ein axiales Scherspiel vorhanden ist, und dass vorzugsweise der Endbereich (**144**) des Innenkörpers (**140**) einen verminderten Durchmesser und/oder eine gerundete oder geschrägte Kante (**145**) aufweist.
